# EUROPEAN PATENT APPLICATION

(11) **EP 2 783 644 A1**
(43) Date of publication of application: **01.10.2014**
(21) Application number: 14161727.4
(22) Date of filing: 26.03.2014
(51) Int. Cl.: A61B 17/3203, F04B 43/04

(54) **Fluid ejection device and medical apparatus**

(30) Priority: 28.03.2013 JP 2013067719
(71) Applicant: Seiko Epson Corporation, Shinjuku-ku, Tokyo 163-0811 (JP)
(72) Inventor: Uchida, Kazuaki, Suwa-shi Nagano, 392-8502 (JP); Karasawa, Junichi, Suwa-shi Nagano, 392-8502 (JP); Matsuzaki, Takahiro, Suwa-shi Nagano, 392-8502 (JP)
(74) Representative: HOFFMANN EITLE

(57) **Abstract**

A fluid ejection device which ejects a fluid includes: an ejection pipe having an opening to eject the fluid; a fluid chamber which communicates with the ejection pipe and changes in volume due to displacement of a piezoelectric element; a supply channel which communicates with the fluid chamber; an opening/closing unit which is provided on the supply channel and opens and closes the supply channel; a fluid supplying unit which pressurizes the fluid and supplies the fluid to the fluid chamber via the supply channel; and a voltage controller which applies a drive voltage to the piezoelectric element. The voltage controller controls the drive voltage applied to the piezoelectric element so that the drive voltage reaches a predetermined voltage after the supply channel is opened by the opening/closing unit.

## Description

### BACKGROUND

### 1. Technical Field

The present invention relates to a fluid ejection device and a medical apparatus.

### 2. Related Art

As a medical apparatus which ejects a fluid to an affected part for treatment, for example, a device disclosed in JP-A-2008-82202 is known. In the fluid ejection device disclosed in JP-A-2008-82202, a piezoelectric element is driven to increase or decrease the volume of a fluid chamber, thus causing a pulsed flow (pulse flow) to be ejected from an ejection pipe.

Since a fluid ejection device is used, for example, as a surgical knife, the force (strength) of a pulsed flow needs to be stable. Particularly in order to improve the sense of use experienced by the operator, there is a demand that a pulsed flow with a proper strength should be ejected from immediately after the start of ejection.

Also, a reduction in size, a reduction in cost, resource saving, easier manufacturing, improvement in usability and the like are demanded of the traditional fluid ejection devices.

### SUMMARY

An advantage of some aspects of the invention is to solve at least a part of the problems described above, and the invention can be implemented as the following aspects.

(1) An aspect of the invention provides a fluid ejection device which ejects a fluid. The fluid ejection device is a fluid ejection device which ejects a fluid and the device includes: an ejection pipe which ejects the fluid; a fluid chamber which communicates with the ejection pipe; a pulsation applying part which causes the fluid in the fluid chamber to be ejected from the ejection pipe; a supply channel which communicates with the fluid chamber; an opening/closing unit which is provided on the supply channel and opens and closes the supply channel; a fluid supplying unit which pressurizes the fluid and supplies the fluid to the fluid chamber via the supply channel; and a voltage controller which applies a drive voltage to the pulsation applying part. The voltage controller controls the drive voltage applied to the pulsation applying part so that the drive voltage reaches a predetermined voltage after the supply channel is opened by the opening/closing unit. Immediately after the supply channel is opened by the opening/closing unit, the pressure of the fluid supplied from the supply channel temporarily rises and the pressure in the fluid chamber temporarily rises, too. According to the fluid ejection device of this aspect, when the supply channel is opened by the opening/closing unit, the drive voltage applied to the pulsation applying part is yet to reach the predetermined voltage. Therefore, application of the predetermined voltage to the pulsation applying part in the state where the pressure of the fluid in the fluid chamber is temporarily high can be restrained. As a result, a pulsed flow with a proper strength can be ejected from immediately after the start of ejection.

(2) In the fluid ejection device of the aspect described above, the voltage controller may perform control to apply the drive voltage to the pulsation applying part after the supply channel is opened by the opening/closing unit. According to the fluid ejection device of this aspect, since the driving of the pulsation applying part is started after the supply of the fluid to the fluid chamber is started, driving of the pulsation applying part in the state where the fluid chamber is short of the fluid can be restrained. As a result, generation of air bubbles due to the driving of the pulsation applying part in the state where the fluid chamber is short of the fluid can be restrained. Therefore, weakening of the propagation of the pressure due to air bubbles can be restrained.

(3) In the fluid ejection device of the aspect described above, the voltage controller may perform control to apply the drive voltage to the pulsation applying part after the lapse of a predetermined time after the supply channel is opened by the opening/closing unit. After the lapse of the predetermined time after the supply channel is opened, the temporarily high pressure in the fluid chamber falls and becomes stable at substantially a constant value. According to the fluid ejection device of this aspect, since the driving of the pulsation applying part is started after the lapse of the predetermined time after the supply channel is opened, a pulsed flow with a proper strength can be ejected from immediately after the start of ejection. Moreover, according to the fluid ejection device of this embodiment, since the driving of the pulsation applying part is started after the supply of the fluid to the fluid chamber is started, generation of air bubbles in the fluid chamber can be restrained and therefore weakening of the propagation of the pressure due to air bubbles can be restrained.

(4) In the fluid ejection device of the aspect described above, the supply channel may include an elastic channel. The opening/closing unit may include a pinch valve which presses the elastic channel from outside and thus closes the supply channel. According to the fluid ejection device of this aspect, since the supply channel can be opened and closed without the opening/closing unit contacting the fluid in the duct, the hygiene of the fluid can be improved.

(5) Another aspect of the invention provides a medical apparatus using the fluid ejection device according to the aspect described above. According to this aspect, a highly reliable medical apparatus can be provided.

Not all of the plural components provided in each of the above aspects of the invention are essential. In order to solve a part or all of the foregoing problems, or in order to achieve a part or all of the advantages described herein, a part of the plural components can be changed, deleted, replaced with another new component, or partly deleted in a limited context. Also, in order to solve a part or all of the foregoing problems, or in order to achieve a part or all of the advantages described herein, a part or all of the technical features included in one aspect of the invention can be combined with a part or all of the technical features of another aspect of the invention, to form a different aspect of the invention.

For example, an aspect of the invention can be implemented as a device having one or more of the following six components: an ejection pipe, a fluid chamber, a supply channel, an opening/closing unit, a fluid supplying unit, and a voltage controller. That is, this device may or may not have an ejection pipe. Similarly, the device may or may not have a fluid chamber. The device may or may not have a supply channel. The device may or may not have an opening/closing unit. The device may or may not have a fluid supplying unit. The device may or may not have a voltage controller. The ejection pipe may be formed, for example, as an ejection pipe having an opening for ejecting the fluid. The fluid chamber may be formed, for example, as a fluid chamber which communicates with the ejection pipe and changes in volume due to displacement of a piezoelectric element. The supply channel may be formed, for example, as a supply channel which communicates with the fluid chamber. The opening/closing unit may be formed, for example, as an opening/closing unit which is provided on the supply channel and opens and closes the supply channel. The fluid supplying unit may be formed, for example, as a fluid supplying unit which pressurizes the fluid and thus supplies the fluid to the fluid chamber via the supply channel. The voltage controller may be formed, for example, as a voltage controller which applies a drive voltage to the piezoelectric element and controls the drive voltage applied to the piezoelectric element so that the drive voltage reaches a predetermined voltage after the supply channel is opened by the opening/closing unit. Such a device can be implemented, for example, as a fluid ejection device which ejects a fluid but can also be implemented as another device than the fluid ejection device which ejects a fluid. According to such an aspect, at least one of the various problems such as a reduction in the size of the device, a reduction in cost, resource saving, easier manufacturing, and improvement in usability can be solved. A part or all of the technical features of each aspect of the foregoing fluid ejection device which ejects a fluid can be applied to this device.

The invention can also be implemented in various other aspects than the device. For example, the invention can be implemented in such aspects as a method for ejecting a fluid and a method for manufacturing a fluid ejection device.

### BRIEF DESCRIPTION OF THE DRAWINGS

The invention will be described with reference to the accompanying drawings, wherein like numbers reference like elements.
FIG. 1 is an explanatory view showing the configuration of a fluid ejection device as an embodiment of the invention.
FIG. 2 is an enlarged cross-sectional view showing a part of the inner configuration of a handpiece.
FIG. 3 is an explanatory view showing the result of measuring the pressure of a fluid in a fluid chamber immediately after a valve is opened.
FIG. 4 is an explanatory view showing a change in the drive voltage applied to a piezoelectric element in a first embodiment.
FIG. 5 is an explanatory view showing an example of a timing chart in the case where a foot switch is turned on.
FIG. 6 is an explanatory view showing a change in the drive voltage applied to the piezoelectric element in a second embodiment.
FIG. 7 is an explanatory view showing an example of a timing chart in the case where the foot switch is turned on in the second embodiment.
FIG. 8 is an explanatory view showing a change in the drive voltage applied to the piezoelectric element in a third embodiment.
FIG. 9 is an explanatory view showing an example of a timing chart in the case where the foot switch is turned on in the third embodiment.
FIG. 10 is a flowchart showing processing in the case where the foot switch is turned on in the third embodiment.

### DESCRIPTION OF EXEMPLARY EMBODIMENTS

Now, embodiments of the invention will be described, in order of first to third embodiments and modifications.

### A. First Embodiment

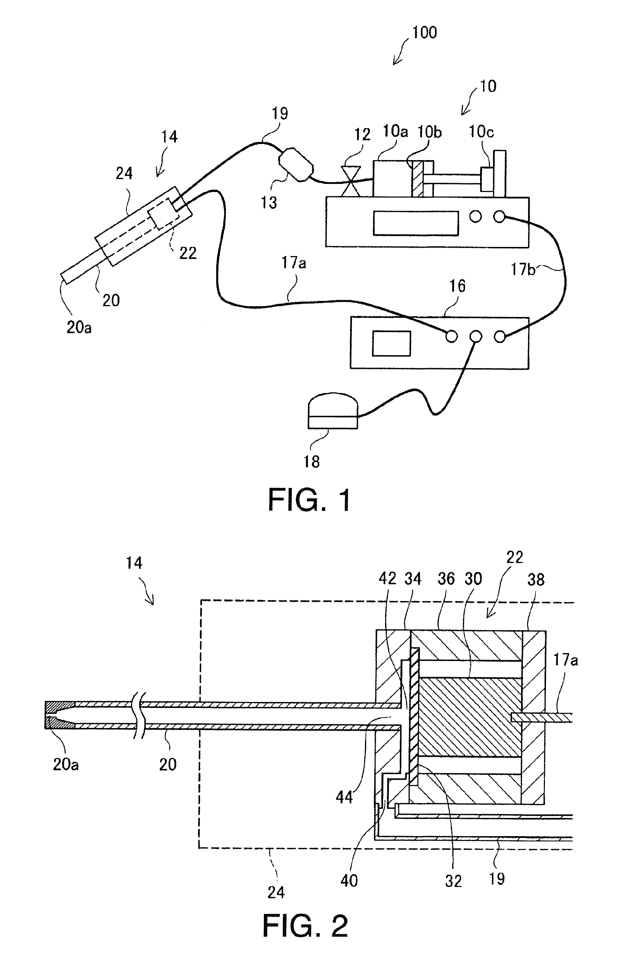

FIG. 1 is an explanatory view showing the configuration of a fluid ejection device 100 as an embodiment of the invention. The fluid ejection device 100 of this embodiment is a medical apparatus used in a medical institution and has the function of a surgical knife which ejects a fluid to an affected part and thereby incises or excises the affect part.

The fluid ejection device 100 has a fluid supplying unit 10, a handpiece 14, a controller 16, and a foot switch 18. The fluid supplying unit 10 and the handpiece 14 are connected to each other by a connection tube 19 made of a resin.

The connection tube 19 is provided with a valve 12 as an opening/closing unit to open and close the channel, and a filter 13 to eliminate foreign matters, bacteria, air bubbles and the like from inside the connection tube 19.

The fluid supplying unit 10 supplies a fluid to the handpiece 14 via the connection tube 19. In this embodiment, the fluid supplying unit 10 is a syringe-type pump, having a cylindrical syringe 10a, a piston 10b which changes the volume of the syringe 10a, and an actuator 10c which moves the piston 10b within the syringe 10a.

The syringe 10a houses a physiological saline solution as a fluid to be supplied to the handpiece 14. However, the syringe 10a may house another fluid that is harmless when ejected to an affected part, for example, pure water, a drug solution or the like, instead of the physiological saline solution.

The piston 10b is movable within the syringe 10a to change the volume of the syringe 10a as the actuator 10c operates. In this embodiment, the piston 10b is made of a resin in order to improve airtightness of the syringe 10a.

The valve 12 is an opening/closing unit which opens and closes the channel. In this embodiment, a pinch valve is used which pinches the elastic connection tube 19 from outside and thus closes the channel inside the connection tube 19. Therefore, the channel can be opened and closed without contacting the fluid in the connection tube 19 and the hygiene of the fluid in the channel can be maintained. Also, the pinch valve can be reused even in the case where the used or old connection tube 19 is disposed of and replaced with a new connection tube 19. However, other types of valves such as gate valve and ball valve may also be used as the valve 12.

In this embodiment, the fluid supplying unit 10 is provided with a sensor which measures the pressure of the fluid in the syringe 10a. In the case where the valve 12 is closed, the actuator 10c is controlled in such a way that the pressure of the fluid in the syringe 10a reaches a predetermined pressure. As the fluid supplying unit 10 receives a command from the controller 16 to supply the fluid to the handpiece 14, the fluid supplying unit 10 opens the valve 12 and causes the actuator 10c to operate, thus moving the piston 10b at a predetermined speed. As a result, the volume of the syringe 10a decreases, extruding the fluid in the syringe 10a into the connection tube 19.

The handpiece 14 is an instrument which the operator holds in the hand and operates. The handpiece 14 has a fluid ejection pipe 20, a pulsation applying part 22, and a casing 24. As a fluid is supplied to the handpiece 14 from the fluid supplying unit 10, pulsation is generated in the supplied fluid by the pulsation applying part 22 and the fluid (pulsed flow) in which pulsation is generated is ejected at a high speed from an opening 20a at the forward end of the fluid ejection pipe 20. The operator applies the fluid ejected from the handpiece 14, to an affected part of a patient, thereby incising or excising the affected part.

The controller 16 applies a drive voltage to the pulsation applying part 22 via a voltage application cable 17a and also controls the fluid supplying unit 10 and the valve 12 via a control cable 17b, thus controlling the flow rate of the fluid supplied to the handpiece 14.

The foot switch 18 is a switch which the operator operates with the foot and is connected to the controller 16. As the operator turns on the foot switch 18, a drive voltage is applied to the pulsation applying part 22, and the valve 12 is opened. Thus, the fluid supplying unit 10 starts supplying the fluid. As a result, the fluid (pulsed flow) in which pulsation is generated is ejected at a high speed from the opening 20a at the forward end of the fluid ejection pipe 20 of the handpiece 14.

In the fluid ejection device 100 of this embodiment, since the supply of the fluid to the handpiece 14 is controlled by opening and closing the valve 12, excellent responsiveness to the operator's operation is achieved.

FIG. 2 is an enlarged cross-sectional view showing a part of the inner configuration of the handpiece 14. Inside the casing 24 of the handpiece 14, the pulsation applying part 22 which applies pulsation to the fluid supplied from the fluid supplying unit 10 is provided. The pulsation applying part 22 has a piezoelectric element 30, a diaphragm 32, a first case 34, a second case 36, and a third case 38.

Inside the pulsation applying part 22, an inlet channel 40, a fluid chamber 42, and an outlet channel 44 are formed as a channel through which the fluid supplied from the fluid supplying unit 10 passes. In this embodiment, the inlet channel 40 and the outlet channel 44 are formed in the first case 34. The fluid chamber 42 is formed between the first case 34 and the diaphragm 32. The connection tube 19 is connected to the inlet channel 40. The fluid ejection pipe 20 is connected to the outlet channel 44.

The diaphragm 32 is a disc-shaped thin metal plate and an outer peripheral part thereof is held and fixed between the first case 34 and the second case 36.

The piezoelectric element 30 changes the volume of the fluid chamber 42 formed between the diaphragm 32 and the first case 34, when a drive voltage is applied to the piezoelectric element 30 from the controller 16. In this embodiment, the piezoelectric element 30 is a multilayer piezoelectric element, with one end thereof fixed to the diaphragm 32 and the other end thereof fixed to the third case 38.

When the drive voltage applied to the piezoelectric element 30 increases, the piezoelectric element 30 expands. The diaphragm 32 is pushed by the piezoelectric element 30 and flexes toward the fluid chamber 42. As the diaphragm 32 flexes toward the fluid chamber 42, the volume of the fluid chamber 42 decreases and the fluid in the fluid chamber 42 is extruded from the fluid chamber 42. In this embodiment, the inner diameter of the outlet channel 44 is greater than the inner diameter of the inlet channel 40. That is, since the inertance of the outlet channel 44 is smaller than the inertance of the inlet channel 40, the majority of the fluid in the fluid chamber 42 is extruded from the fluid chamber 42 through the outlet channel 44.

Meanwhile, when the drive voltage applied to the piezoelectric element 30 decreases, the piezoelectric element 30 contracts and the volume of the fluid chamber 42 increases. Thus, the fluid is supplied into the fluid chamber 42 from the inlet channel 40.

Since the drive voltage applied to the piezoelectric element repeats on-state (maximum voltage) and off-state (0 V) at a high frequency (for example, 300 Hz), an increase and decrease in the volume of the fluid chamber 42 is repeated, thus generating pulsation in the fluid. The fluid extruded from the fluid chamber 42 is ejected from the nozzle 20a (opening 20a) at the forward end of the fluid ejection pipe 20. While the off-voltage is described as 0 V, the off-voltage need not be 0 V and may be any voltage that is lower than the maximum voltage of the on-state.

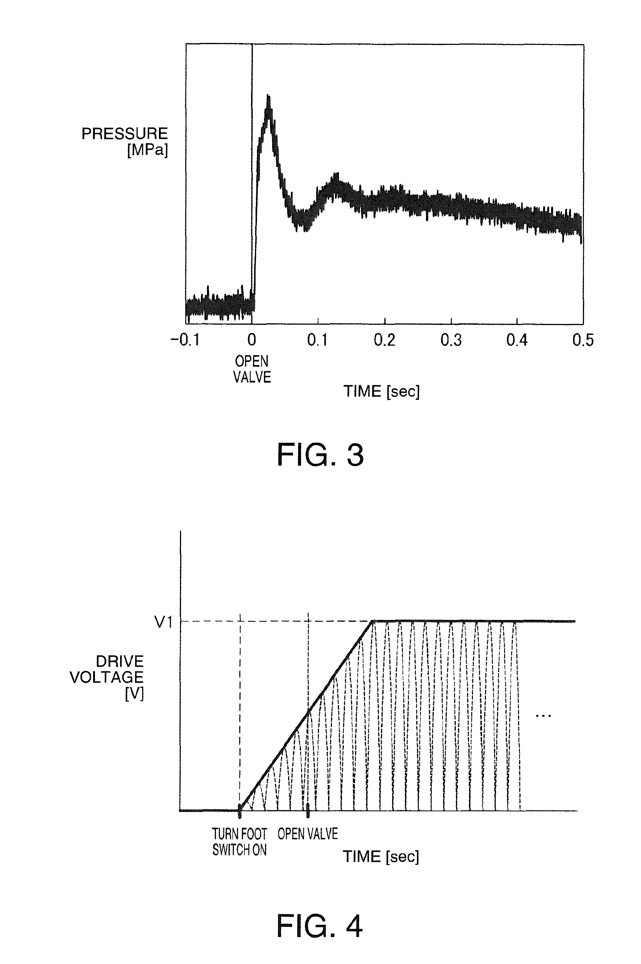

FIG. 3 is an explanatory view showing the result of measuring the pressure of the fluid in the fluid chamber 42 immediately after the valve 12 is opened. In FIG. 3, the horizontal axis represents time and the vertical axis represents the pressure of the fluid in the fluid chamber 42. The piezoelectric element 30 is not driven in this measurement of the pressure shown in FIG. 3.

As shown in FIG. 3, it is confirmed that as the valve 12 is opened and the fluid supplying unit 10 starts supplying the fluid at time 0, the pressure of the fluid in the fluid chamber 42 temporarily takes a high value immediately after the opening of the valve 12 and then falls and becomes stable at a substantially constant value.

One of the reasons for this can be considered as follows. When the valve 12 is opened in the state where a high pressure is applied to the syringe 10a, the fluid is urged to flow out straightaway to the handpiece 14. However, in the course of the channel from the fluid supplying unit 10 to the fluid chamber 42 of the handpiece 14, an element that causes channel resistance such as the filter 13 exists, thus temporarily damming up the fluid. Meanwhile, since the supply of the fluid from the syringe 10a is continued, the pressure temporarily rises at the point before the resistance element such as the filter 13 and this high pressure flows into the handpiece 14. Moreover, it is also conceivable that the opening of the valve 12 is regarded as a step input in the process of pressure transmission and therefore a high pressure is generated in the fluid chamber 42 of the handpiece 14 immediately after the opening of the valve 12.

FIG. 4 is an explanatory view showing a change in the drive voltage applied to the piezoelectric element 30 in the first embodiment. The broken lines shown in FIG. 4 represent an example of the drive voltage applied to the piezoelectric element 30. In practice, the drive voltage applied to the piezoelectric element 30 repeats on-state (maximum voltage) and off-state (0 V) at a high frequency (for example, 300 Hz). However, the drive voltage is shown at a lower frequency than the actual frequency in order to make the transition of the maximum voltage easier to understand. The solid lines in FIG. 4 show the transition of the maximum voltage of the drive voltage. The scale on the horizontal axis in FIG. 4 is different from FIG. 3. Hereinafter, in the drawings showing a change in the drive voltage applied to the piezoelectric element 30, only the transition of the maximum voltage of the drive voltage is shown.

As shown in FIG. 4, when the foot switch 18 is turned on, the controller 16 starts application of the drive voltage to the piezoelectric element 30 and controls the maximum voltage of the drive voltage to gradually increase and reach a predetermined voltage V1. The controller 16 also opens the valve 12 and causes the actuator 10c of the fluid supplying unit 10 to operate, thus starting to supply the fluid.

In this embodiment, the controller 16 controls the maximum voltage of the drive voltage applied to the piezoelectric element 30 to reach the predetermined voltage V1 after the valve 12 is opened. Therefore, as shown in FIG. 4, the maximum voltage of the drive voltage is yet to reach the predetermined voltage V1 when the valve 12 is opened.

Specifically, in this embodiment, a lower drive voltage than the predetermined voltage V1 is applied to the piezoelectric element 30 immediately after the valve 12 is opened, that is, when the pressure of the fluid in the fluid chamber 42 is temporarily high. After the pressure of the fluid in the fluid chamber 42 is stabilized at a substantially constant value, the drive voltage having the predetermined voltage V1 as the maximum voltage is applied to the piezoelectric element 30. Therefore, ejection of a pulsed flow with a great strength immediately after the start of ejection can be restrained. According to this embodiment, a pulsed flow with a proper strength can be ejected from immediately after the start of ejection.

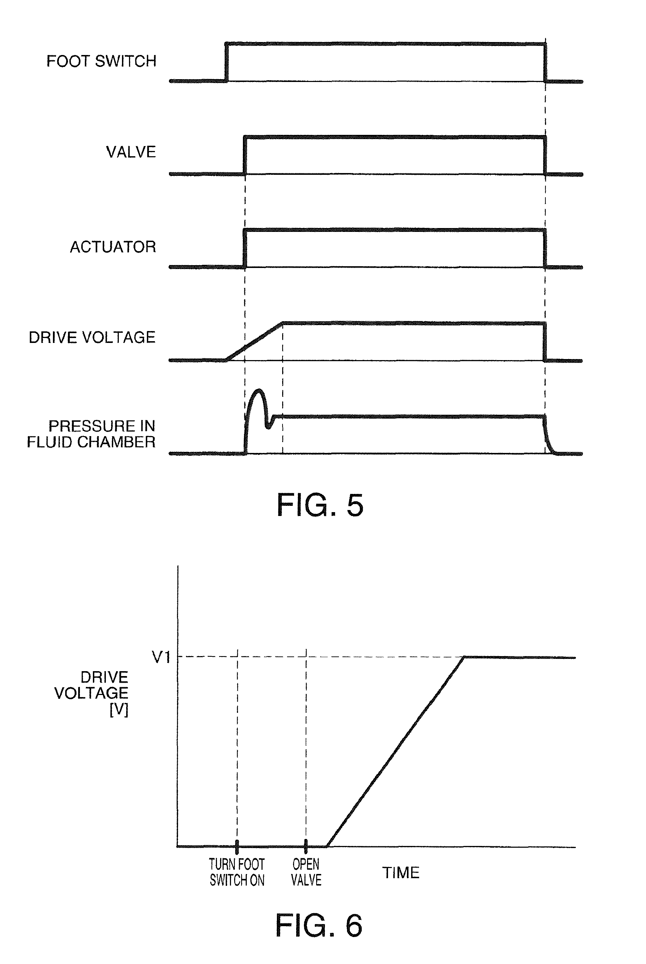

FIG. 5 is an explanatory view showing an example of a timing chart in the case where the foot switch 18 is turned on. Triggered by the turning on of the foot switch 18, the controller 16 starts to apply the drive voltage. Also, triggered by the turning on of the foot switch 18, the controller 16 opens the valve 12 and causes the actuator 10c to operate. Thus, the pressure of the fluid in the fluid chamber 42 temporarily rises immediately after the opening of the valve 12, and then becomes stable at a substantially constant value. As described above, in this embodiment, during the period when the pressure of the fluid in the fluid chamber 42 is temporarily high, a lower drive voltage than the predetermined voltage V1 is applied to the piezoelectric element 30, and after the pressure of the fluid in the fluid chamber 42 is stabilized at a substantially constant value, a drive voltage having the predetermined voltage V1 as the maximum voltage is applied to the piezoelectric element 30.

Meanwhile, triggered by the turning off of the foot switch 18, the controller 16 closes the valve 12 and stops the actuator 10c to stop the application of the drive voltage.

If the time until the maximum voltage of the drive voltage reaches the predetermined voltage V1 from the turning on of the foot switch 18 is shorter, the sense of use experienced by the operator can be improved. Therefore, it is preferable that the time until the maximum voltage of the drive voltage reaches the predetermined voltage V1 from the turning on of the foot switch 18 is 0.2 seconds or shorter.

In this way, according to this embodiment, a lower drive voltage than the predetermined voltage V1 is applied to the piezoelectric element 30 immediately after the valve 12 is opened, that is, when the pressure of the fluid in the fluid chamber 42 is temporarily high. Therefore, a pulsed flow with a proper strength can be ejected from immediately after the start of ejection.

### B. Second Embodiment

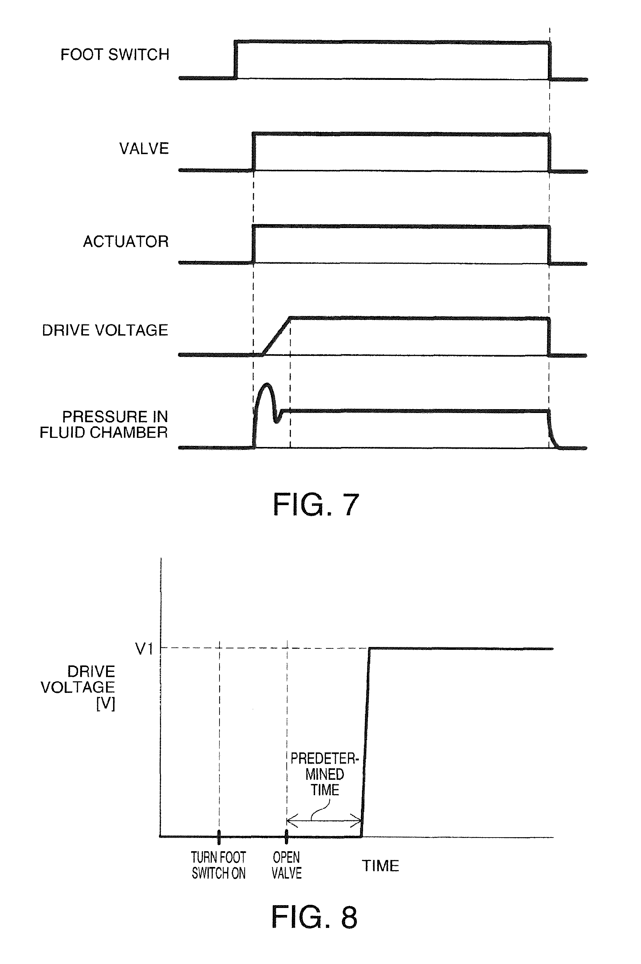

FIG. 6 is an explanatory view showing a change in the maximum voltage of the drive voltage applied to the piezoelectric element 30 in a second embodiment. FIG. 7 is an explanatory view showing an example of a timing chart in the case where the foot switch 18 is turned on in the second embodiment. The second embodiment is different from the first embodiment shown in FIGS. 4 and 5, only in that the drive voltage is applied to the piezoelectric element 30 after the valve 12 is opened. The other configurations are the same as in the first embodiment.

According to this embodiment, since the driving of the piezoelectric element 30 is started after the supply of the fluid to the fluid chamber 42 is started, driving of the piezoelectric element 30 in the state where the fluid chamber 42 is short of the fluid can be restrained. As a result, generation of air bubbles due to the driving of the piezoelectric element 30 in the state where the fluid chamber 42 is short of the fluid can be restrained.

In this way, according to this embodiment, similar effects to those of the first embodiment can be achieved. Also, generation of air bubbles in the fluid chamber 42 can be restrained and therefore weakening of the propagation of the pressure due to air bubbles can be restrained.

### C. Third Embodiment

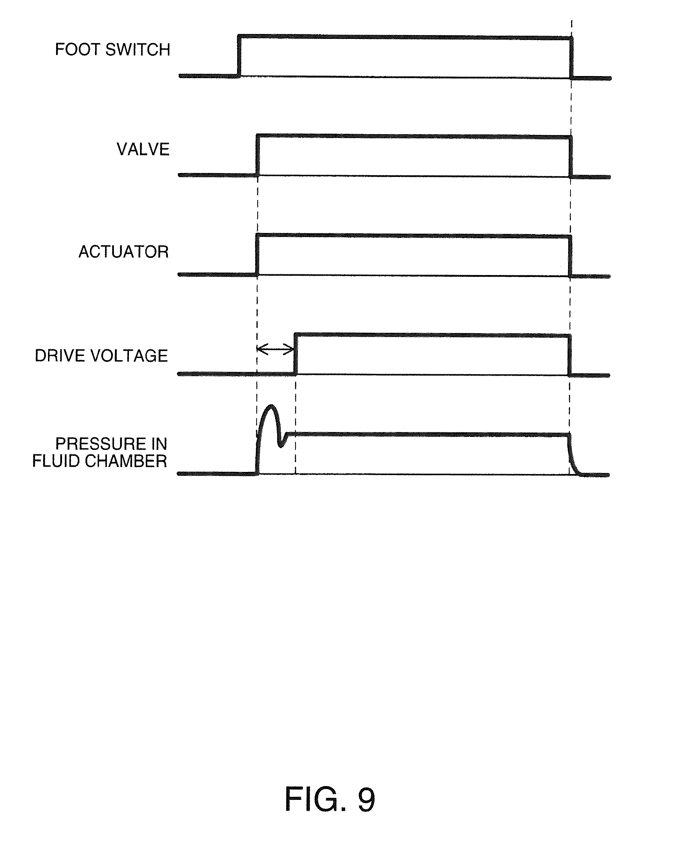

FIG. 8 is an explanatory view showing a change in the maximum voltage of the drive voltage applied to the piezoelectric element 30 in a third embodiment. FIG. 9 is an explanatory view showing an example of a timing chart in the case where the foot switch 18 is turned on in the third embodiment. The third embodiment is different from the first embodiment shown in FIGS. 4 and 5, only in that the drive voltage is applied to the piezoelectric element 30 after the lapse of a predetermined time after the valve 12 is opened and that the maximum voltage of the drive voltage is already the predetermined voltage V1 immediately after the start of the application. The other configurations are the same as in the first embodiment.

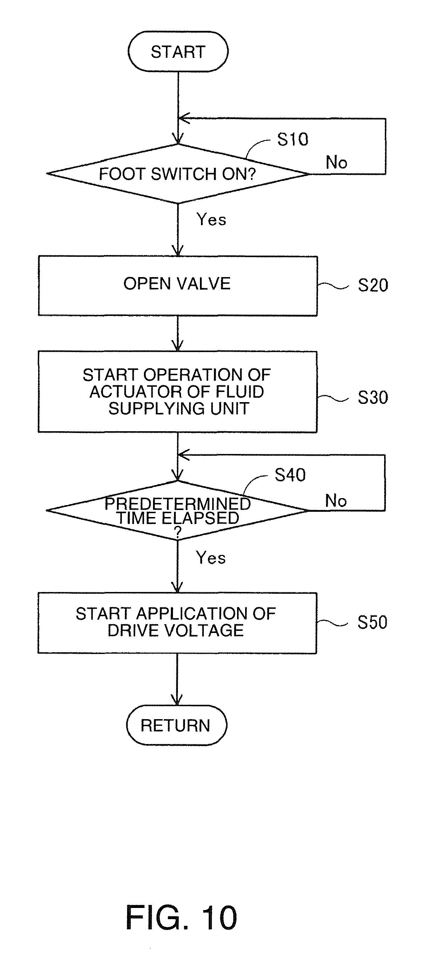

FIG. 10 is a flowchart showing processing in the case where the foot switch 18 is turned on in the third embodiment. The controller 16 determines whether the foot switch 18 is on or not (step S10). If the foot switch 18 is on, the controller 16 opens the valve 12 (step S20) and then causes the actuator 10c of the fluid supplying unit 10 to operate (step S30). The controller 16 determines whether a predetermined time has elapsed from the opening of the valve 12 or not (step S40). If the predetermined time has elapsed, the controller 16 starts application of a drive voltage to the piezoelectric element 30 (step S50).

As shown in FIGS. 3 and 9, after the lapse of the predetermined time after the valve 12 is opened, the temporarily high pressure in the fluid chamber 42 falls and becomes stable at a substantially constant value. In this embodiment, the piezoelectric element 30 starts to be driven after the lapse of the predetermined time after the valve 12 is opened, that is, after the temporarily high pressure in the fluid chamber 42 falls and becomes stable at a substantially constant value. Therefore, a pulsed flow with a proper strength can be ejected from immediately after the start of ejection.

Moreover, in this embodiment, since the piezoelectric element 30 starts to be driven after the valve 12 is opened and the supply of the fluid to the fluid chamber 42 is started, driving of the piezoelectric element 30 in the state where the fluid chamber 42 is short of the fluid can be restrained. As a result, generation of air bubbles due to driving of the piezoelectric element 30 in the state where the fluid chamber 42 is short of the fluid can be restrained and therefore weakening of the propagation of the pressure due to air bubbles can be restrained.

According to FIG. 3, in can be understood that in the fluid ejection device 100 of this embodiment, the pressure of the fluid in the fluid chamber 42 becomes stable at a substantially constant value approximately 0.1 seconds after valve 12 is opened. Therefore, it is preferable that the controller 16 in this embodiment controls the drive voltage to be applied to the piezoelectric element 30 approximately 0.1 seconds after the valve 12 is opened. However, the time taken for the pressure of the fluid in the fluid chamber 42 to become stable at a substantially constant value varies depending on the configuration of the fluid ejection device 100. Therefore, it is preferable that the time until the application of the drive voltage is started after the valve 12 is opened is properly set according to the configuration of the fluid ejection device 100.

### D. Modifications

The invention is not limited to the above embodiments and can be carried out in various forms without departing from the scope of the invention. For example, the following modifications can be made.

### Modification 1

In the embodiments, the fluid ejection device 100 is used as a medical apparatus. However, as a modification, the fluid ejection device 100 may be used as another apparatus than the medical apparatus. For example, the fluid ejection device 100 may be used as a cleaning device which ejects a fluid to a target object and thus removes stains from the target object, or a drawing device which draws letters, pictures, and the like with the ejected fluid.

### Modification 2

In the embodiments, a liquid is used as a fluid ejected from the fluid ejection device 100. However, as a modification, a gas may be used as a fluid ejected from the fluid ejection device 100.

### Modification 3

In the embodiments, the controller 16 may have, for example, a CPU and a DAC (digital-analog conversion circuit) and may cause a drive waveform shaped by the CPU to be converted into an analog drive signal by the DAC and then supply the drive signal to the piezoelectric element 30. Also, a part of the functions implemented by software in the embodiments may be implemented by hardware, or a part of the functions implemented by hardware may be implemented by software.

### Modification 4

In the second and third embodiments, the timing of opening the valve 12 and starting the operation of the actuator 10c may be immediately after the foot switch 18 is turned on. Thus, the time until the maximum voltage of the drive voltage reaches the predetermined voltage V1 can be reduced.

### Modification 5

In the embodiments, a switch operated with the hand may be provided instead of the foot switch 18 operated with the foot. The switch operated with the hand may be provided, for example, on the handpiece 14.

### Modification 6

In the embodiments, a piezoelectric element is used as the pulsation applying part. However, as a modification 6, an air bubble generator may be used as the pulsation applying part. As the air bubble generator, for example, a heater, laser beam casting unit or the like may be used. As the air bubble generator, any unit that heats the fluid in the fluid chamber and thus generates air bubbles so that the fluid in the fluid chamber is ejected by expansion of the generated air bubbles can be used.

The invention is not limited to the above embodiments, examples and modifications and can be implemented in various configurations without departing from the scope of the invention. For example, the technical features in the embodiments, examples and modifications corresponding to the technical features in the respective embodiments described in the summary of the invention can be suitably replaced or combined in order to solve a part or all of the foregoing problems or in order to achieve a part or all of the foregoing advantages. Also, the technical features can be suitably deleted unless these features are described as essential herein.

## Claims

1. A fluid ejection device which ejects a fluid, comprising:
an ejection pipe which ejects the fluid;
a fluid chamber which communicates with the ejection pipe;
a pulsation applying part which causes the fluid in the fluid chamber to be ejected from the ejection pipe;
a supply channel which communicates with the fluid chamber;
an opening/closing unit which is provided on the supply channel and opens and closes the supply channel;
a fluid supplying unit which pressurizes the fluid and supplies the fluid to the fluid chamber via the supply channel; and
a voltage controller which applies a drive voltage to the pulsation applying part;
wherein the voltage controller controls the drive voltage applied to the pulsation applying part so that the drive voltage reaches a predetermined voltage after the supply channel is opened by the opening/closing unit.

2. The fluid ejection device according to claim 1, wherein the voltage controller performs control to apply the drive voltage to the pulsation applying part after the supply channel is opened by the opening/closing unit.

3. The fluid ejection device according to claim 1 or 2, wherein the voltage controller performs control to apply the drive voltage to the pulsation applying part after the lapse of a predetermined time after the supply channel is opened by the opening/closing unit.

4. The fluid ejection device according to any one of the preceding claims, wherein the supply channel includes an elastic channel, and
the opening/closing unit includes a pinch valve which presses the elastic channel from outside and thus closes the supply channel.

5. A medical apparatus using the fluid ejection device according to any one of the preceding claims.

6. A medical apparatus using the fluid ejection device according to claim 2.

7. A medical apparatus using the fluid ejection device according to claim 3.

8. A medical apparatus using the fluid ejection device according to claim 4.
